(19) Europäisches Patentamt

European Patent Office

Office européen des brevets

(11) **EP 0 792 863 B1**

(12) **FASCICULE DE BREVET EUROPEEN**

(45) Date de publication et mention
de la délivrance du brevet:
**27.06.2001 Bulletin 2001/26**

(51) Int Cl.⁷: **C07C 45/74**, C07C 49/603,
B01J 21/10

(21) Numéro de dépôt: **97400120.8**

(22) Date de dépôt: **21.01.1997**

(54) **Procédé d'obtention d'isophorone**

Verfahren zur Herstellung von Isophoron

Process for producing isophorone

(84) Etats contractants désignés:
**BE DE ES FR GB IT NL**

(30) Priorité: **29.02.1996 FR 9602550**

(43) Date de publication de la demande:
**03.09.1997 Bulletin 1997/36**

(73) Titulaire: **Atofina**
**92800 Puteaux (FR)**

(72) Inventeurs:
• **Teissier, Remy**
**69340 Francheville (FR)**
• **Kervennal, Jacques**
**69005 Lyon (FR)**

(56) Documents cités:
**EP-A- 0 095 783        EP-A- 0 640 387**
**WO-A-90/12645        WO-A-92/00266**
**US-A- 5 254 743**

## Description

La présente invention concerne un nouveau procédé d'obtention d'isophorone à partir de l'acétone.

L'isophorone est utilisée industriellement comme solvant et est également un important intermédiaire de synthèse , notamment pour la fabrication du 3,5-xylénol. L'autocondensation de l'acétone en isophorone peut être effectuée soit en phase liquide soit en phase vapeur. Le procédé en phase liquide utilise en général des solutions diluées d'hydroxyde de potassium ou de sodium comme catalyseur (GB 1 528 129, BE 611 719, US 2 399 976, FR 1 506 158). Ce procédé présente toutefois de nombreux inconvénients. Les catalyseurs ne sont pas très sélectifs. Une partie seulement des catalyseurs est recyclée après décantation et séparation de l'isophorone. L'autre partie non recyclée est neutralisée par de l'acide sulfurique et par conséquent conduit à des sels, dont l'élimination nécessaire pour l'environnement est coûteuse.

Par ailleurs, l'autocondensation de l'acétone en phase vapeur s'opère à température élevée, en général supérieure à 200° C et en présence de catalyseurs basiques tels que les oxydes de magnésium ou d'aluminium (K.V. Ramanamurthy et al. Proc. 8th Nat. Symp. Catal., Sindri, India, 12-14 Feb. 1987, p. 649), les oxydes mixtes à base de magnésium et d'aluminium (EP 640 387, US 4 970 191). La demande de brevet EP 95783 décrit l'utilisation d'hydrotalcite synthétique calcinée à une température comprise entre 300°C et 600°C pour l'autocondensation de l'acétone en phase vapeur à température élevée (300°C). Les inconvénients de cette réaction en phase vapeur sont nombreux. Ainsi la préparation des catalyseurs est peu reproductible et leur mise en forme pour éviter les phénomènes diffusionnels n'est pas très aisée (US 5 153 166). De plus, la durée de vie de ces catalyseurs est réduite par le dépôt de coke à la surface qui s'accompagne de sous-produits lourds non recyclables.

Il a maintenant été trouvé un nouveau procédé d'obtention d'isophorone à partir de l'acétone, qui a l'avantage de pouvoir utiliser les installations existantes opérant en phase liquide sans pour autant présenter les inconvénients précités. Ce procédé est caractérisé en ce que l'on opère soit en phase gazeuse soit en phase liquide en présence d'un catalyseur de formule générale (I):

$$[ (Mg^{2+})_{1-x} (Al^{3+})_x (OH^-)_2 ]^{x+} [ (OH^-)_x ]^{x-} (H_2O)_n \qquad (I)$$

avec $0,20 \le x \le 0,33$ et $0 < n < 1$.

Le catalyseur de formule générale (I) a une structure lamellaire similaire à celle de l'hydrotalcite.

Comme l'hydrotalcite naturelle, ce catalyseur se compose de couches du type brucite chargées positivement de formule $[ Mg_{1-x} Al_x (OH)_2 ]$ avec $0,2 \le x \le 0,33$ et d'inter-couches constituées d'hydroxydes OH- et de molécules d'eau.

On utilise de préférence le catalyseur de formule générale (I) ayant une valeur de n pouvant aller de 0,5 à 0,75.

Avantageusement, on choisit pour la réaction d'autocondensation de l'acétone en isophorone un catalyseur de formule générale (I) pour laquelle la valeur de n est égale à 0,81 - x, notamment la Meixnerite de formule :

$$[ (Mg^{2+})_{0,75} (Al^{3+})_{0,25} (OH^-)_2]^{0,25+} [(OH^-)_{0,25}]^{0,25-} (H_2O)_{0,5}$$

Le catalyseur de formule générale (I) peut être préparé suivant la méthode décrite par G. Mascolo et O. Marino dans Mineralogical Magazine, March 1980, vol. 43 p. 619.

Cette méthode de préparation consiste à mettre en suspension du gel d'alumine et du MgO, obtenu par calcination de carbonate basique de magnésium à 650° C, dans de l'eau distillée dans un récipient fermé en Téflon, sous agitation, pendant une semaine à 80 ± 1° C. La suspension est ensuite filtrée à l'abri du $CO_2$ et enfin le solide recueilli est séché sur silicagel.

Ce catalyseur peut également être préparé par hydratation d'un double oxyde de magnésium et d'aluminium de formule $Mg_{1-x} Al_x O_{1+x/2}$ en absence de $CO_2$. L'hydratation est effectuée par de l'eau soit en phase liquide soit en phase vapeur. Le double oxyde mixte peut être soit un produit commercial soit le produit obtenu par calcination des hydrotalcites ayant une valeur de x pouvant aller de 0,2 à 0,33.

Après l'étape d'hydratation suivant l'une quelconque des méthodes décrites ci-dessus, le solide peut être séché soit par évaporation à pression réduite à une température inférieure à 60° C soit par rinçage avec un solvant miscible à l'eau, comme par exemple l'acétone.

Pour préparer le catalyseur de formule générale (I), on choisit avantageusement un double oxyde commercial et de préférence celui de la société japonaise KYOWA référencé KW2000 ayant une valeur de x voisine de 0,3.

Le double oxyde est le plus souvent hydraté en phase liquide et le solide ainsi obtenu est avantageusement rincé avec un solvant miscible à l'eau et de préférence avec de l'acétone.

Bien que le catalyseur de formule générale (I) puisse être mis en oeuvre dans la réaction d'autocondensation de l'acétone en isophorone en phase vapeur, on préfère l'utiliser dans la réaction en phase liquide..

Le procédé d'obtention d'isophorone à partir de l'acétone suivant la présente invention peut être effectué en continu ou en discontinu. Industriellement on préfère opérer en continu.

Lorsqu'on opère en continu, le catalyseur peut être soit placé dans un lit fixe et traversé par de l'acétone liquide, soit mis en contact avec de l'acétone liquide dans un lit agité.

Quelle que soit la façon d'opérer (en continu ou en discontinu), la pression doit être suffisante pour maintenir l'acétone en phase liquide à la température de l'autocondensation. On utilise en général une température comprise entre environ 100° C et environ 250° C et le plus souvent une température comprise entre environ 110° C et environ 220° C.

En discontinu, la durée de la réaction peut aller de 30 minutes à 8 heures et de préférence d'une heure à 4 heures. Mais on ne sortira pas du cadre de la présente invention pour une durée de réaction supérieure à 8 heures.

A la fin de la réaction, l'isophorone est séparé des sous-produits par des techniques classiques (distillation, décantation). Les sous-produits comprenant essentiellement l'oxyde de mésityle, le diacétone alcool et des composés en $C_{12}$ et en $C_{15}$ peuvent être recyclés, et donc valorisés.

L'invention sera mieux comprise à partir des exemples suivants :

**I) Partie expérimentale**

1) Préparation de catalyseur

On hydrate par de l'eau en phase liquide le double oxyde mixte KW 2000 ayant les caractéristiques suivantes :

Formule chimique : 4,5 MgO. $Al_2 O_3$ (x = 0,3077)
BET = 172 $m^2$/g
Taille moyenne de particule : 70 $\mu$m
Propriété d'absorption : absorbe au maximum 70-80 parties d'eau pour 100 parties de KW 2000

Ainsi 6 grammes de KW 2000 sont ajoutés sous agitation à 200 ml d'eau décarbonatée (eau permutée, puis bouillie). On laisse le mélange pendant 3 heures sous agitation, puis on sépare le solide. Le solide isolé est ensuite rincé plusieurs fois à l'acétone avant d'être conservé à l'abri de $CO_2$.On obtient 9 g de solide de formule générale (I) où x vaut 0,3077 et qui a une structure cristalline de type de l'hydrotalcite ou de la Meixnerite.

2) Test catalytique

Mode opératoire générale.

Dans un autoclave de 0,5 litre, agité et thermostaté, on introduit à température ambiante environ 50 à 200 g d'acétone et une quantité de catalyseur solide comprise entre environ 1 g à 10 g. L'autoclave est ensuite fermé, puis purgé à l'azote.et éventuellement une pression d'azote jusqu' à environ 20 bars peut être appliquée. On démarre l'agitation pour atteindre une vitesse d'environ 500 à 1 500 tours par minute. Le mélange est ensuite porté à une température comprise entre 110° C et 220° C en une période allant de 30 minutes à 2 heures. Puis la température est maintenue pendant une durée comprise entre 1 heure et 4 heures. A la fin de la réaction, l'autoclave est refroidi rapidement en environ 5 à 10 minutes à l'aide d'une circulation d'eau. Après refroidissement, on ouvre l'autoclave et on sépare le catalyseur de la solution finale par simple filtration ou décantation. La solution finale contenant de l'acétone, du diacétonealcool, d'oxyde de mésityle, de l'isophorone, les composés en $C_{12}$ et en $C_{15}$ est ensuite analysée à l'aide de la chromatographie en phase gazeuse.

Conditions chromatographiques

Chromatographe Hewlett-Packard 5710 muni d'une colonne HP1 de longueur de 30 m et de diamètre 0,53 mm La température de l'injecteur est de 150° C et la température de détecteur FID est de 200° C.

Le four est programmé de façon à maintenir sa température à 60° C pendant 6 minutes et ensuite à augmenter sa température avec une vitesse de chauffe de 8° C par minute jusqu'à atteindre 250° C.

Le four est programmé de façon à maintenir sa température à 60° C pendant 6 minutes et ensuite à augmenter sa température avec une vitesse de chauffe de 8° C par minute jusqu'à atteindre 250° C.

La conversion de l'acétone est définie par la formule suivante :

$$\frac{100 \times [ \text{(nombre de moles d'acétone)}_o - \text{(nombre de moles d'acétone)}_f ]}{\text{(nombre de moles d'acétone)}_o}$$

avec (nombre de moles d'acétone)$_o$ = nombre de moles d'acétone introduits dans le réacteur.
(nombre de moles d'acétone)$_f$ = nombre de moles d'acétone restants à la fin dans la réaction.
La sélectivité en isophorone est définie par la formule suivante :

$$\frac{300 \times [ ( \text{nombre de moles d'ipho)}_f ]}{\text{(nombre de moles d'acétone)}_o - \text{(nombre de moles d'acétone)}_f}$$

avec (nombre de moles d'ipho)$_f$ = nombre de moles d'isophorone formés à la fin de la réaction.

3) ***Exemples***

**Exemple 1.** (non conforme à l'invention)

Dans un autoclave de 0,5 litre, agité et thermostaté, on introduit à température ambiante 3 g de catalyseur KW 2000 et 100 g d'acétone. On ferme l'autoclave, puis on purge à l'azote et on démarre l'agitation. Le mélange est ensuite porté en une heure à la température de 150° C sous une vitesse d'agitation de 1000 tours par minute. Le mélange agité est maintenu à 150° C pendant une heure et la pression du milieu réactionnel est alors à environ 10 bars. Au bout d'une heure de réaction à 150° C, l'autoclave est refroidi en 5 minutes à l'aide d'une circulation d'eau. On ouvre ensuite l'autoclave refroidi et le catalyseur est séparé du mélange final, par simple filtration. La composition de la solution finale est déterminée à l'aide de la chromatographie phase gaz.
Le mélange final est composé en % massique de :

| | |
|---|---|
| Acétone | 75 |
| Oxyde de mésityle | 9 |
| Diacétone alcool | 3 |
| Isophorone | 7,3 |
| Composés en $C_{12}$ | 1 |
| Composés en $C_{15}$ | 0,8 |
| Composés non identifiés + eau | 3,9 |

Ceci correspond à une conversion de l'acétone de 25 % et une sélectivité de 37 % en isophorone. La sélectivité totale en $C_{12}$, oxyde de mésityle, diacétone alcool et isophorone est de 97 %.

***Exemple 2.*** selon l'invention.

On opère de manière identique à l'exemple 1 sauf qu'à la place du catalyseur KW 2000, on utilise 3 g de catalyseur hydraté et rincé suivant la méthode décrite en I.1). On introduit également 105 g d'acétone au lieu de 100 g et on maintient l'autoclave à 200°C au lieu de 150°C. La pression du milieu réactionnel est de 25 bars.
La composition de la solution finale est reportée dans le Tableau 1.
On obtient une conversion de l'acétone de 38 % et une sélectivité en isophorone de 51 %.

***Exemple 3.*** selon l'invention.

On opère de manière identique à l'exemple 2 sauf que la température est maintenue à 120° C pendant 4 heures au lieu de 200° C pendant une heure. On introduit également dans le réacteur 104 g d'acétone au lieu de 105 g. La composition de la solution finale est reportée dans le Tableau 1.
On obtient une conversion de l'acétone de 25 % et une sélectivité en isophorone de 24 %.

***Exemple 4.*** selon l'invention.

On opère de manière identique à l'exemple 3 sauf que l'autoclave est maintenu à une température de 150° C au lieu de 120° C et que l'on introduit 103 g d'acétone au lieu de 104 g.

La composition de la solution finale est reportée dans le Tableau 1.

On obtient une conversion de l'acétone de 31 % et une sélectivité en isophorone de 45 %.

**Exemple 5.** selon l'invention.

On opère de manière identique à l'exemple 1 mais à la place de KW 2000, on introduit le catalyseur hydraté et rincé suivant la méthode décrite en 1.1. Au bout d'une heure de réaction à 150° C, on obtient une conversion de l'acétone de 27 % et une sélectivité en isophorone de 48 %.

Dans les conditions expérimentales identiques le double oxyde est moins sélectif que le catalyseur hydraté de formule générale (I)

| Exemples | Composition finale % massique | | | | | | |
|---|---|---|---|---|---|---|---|
| | Acétone | Oxyde de mésityle | Diacétone Alcool | Isophorone | Composés en | | Composés non identifiés + eau |
| | | | | | $C_{12}$ | $C_{15}$ | |
| 1 (NC)* | 75 | 9 | 3 | 7,3 | 1 | 0,8 | 3,9 |
| 2 (C)* | 61 | 8 | 3 | 14,8 | 2,4 | 3,7 | 7,1 |
| 3 (C) | 75 | 12,7 | 4 | 4,5 | 0,4 | 0,2 | 3,2 |
| 4 (C) | 69 | 8,2 | 3 | 12 | 1,1 | 1,3 | 5,4 |

Tableau 1

*Dans ce tableau   NC signifie : non conforme à l'invention
C signifie : selon l'invention

EP 0 792 863 B1

**Revendications**

1. Procédé d'obtention d'isophorone à partir de l'acétone caractérisé en ce que l'on opère soit en phase gazeuse soit en phase liquide en présence d'un catalyseur de formule générale (I):

$$[\,(Mg^{2+})_{1-x}\,(Al^{3+})_x\,(OH^-)_2\,]^{x+}\,[\,(OH^-)_x\,]^{x-}\,(H_2O)_n \qquad (I)$$

avec $0,20 \leq x \leq 0,33$ et $0 < n < 1$.

2. Procédé selon la revendication 1 caractérisé en ce que la valeur de n est comprise entre 0,5 et 0,75, les limites incluses.

3. Procédé selon la revendication 1 ou 2 caractérisé en ce que la valeur de n est égale à 0,81 - x.

4. Procédé suivant l'une quelconque des revendications 1 à 3 caractérisé en ce que l'on opère en phase liquide à une température comprise entre 100° C et 250° C.

5. Procédé suivant la revendication 4 caractérisé en ce que la température est comprise entre 110° C et 220° C.


**Patentansprüche**

1. Verfahren zur Herstellung von Isophoron aus Aceton, dadurch gekennzeichnet, daß man entweder in Gasphase oder in flüssiger Phase in Gegenwart eines Katalysators der allgemeinen Formel (I) arbeitet:

$$[(Mg^{2+})_{1-x}\,(Al^{3+})_x\,(OH^-)_2]^{x+}\,[(OH^-)_x]^{x-}\,(H_2O)_n \qquad (I)$$

mit $0,20 \leq x \leq 0,33$ und $0 < n < 1$.

2. Verfahren nach Anspruch 1, dadurch gekennzeichnet, daß der Wert von n zwischen 0,5 und 0,75 liegt, die Grenzen eingeschlossen.

3. Verfahren nach Anspruch 1 oder 2, dadurch gekennzeichnet, daß der Wert von n gleich 0,81 - x ist.

4. Verfahren nach irgendeinem der Ansprüche 1 bis 3, dadurch gekennzeichnet, daß man in flüssiger Phase bei einer Temperatur zwischen 100 °C und 250 °C arbeitet.

5. Verfahren nach Anspruch 4, dadurch gekennzeichnet, daß die Temperatur zwischen 110 °C und 220 °C liegt.


**Claims**

1. Process for obtaining isophorone from acetone, characterized in that the operation is carried out either in gaseous phase or in liquid phase in the presence of a catalyst of general formula (I) :

$$[(Mg^{2+})_{1-x}(Al^{3+})_x(OH^-)_2]^{x+}\,[(OH^-)_x]^{x-}(H_2O)_n \qquad (I)$$

with $0.20 \leq x \leq 0.33$ and $0 < n < 1$.

2. Process according to Claim 1, characterized in that the value of n is between 0.5 and 0.75, the limits included.

3. Process according to Claim 1 or 2, characterized in that the value of n is equal to 0.81 - x.

4. Process in accordance with any one of Claims 1 to 3, characterized in that the operation is carried out in liquid phase at a temperature of between 100°C and 250°C.

5. Process in accordance with Claim 4, characterized in that the temperature is between 110°C and 220°C.